# EUROPÄISCHE PATENTSCHRIFT

(11) **EP 1 426 016 B1**
(45) Veröffentlichungstag und Bekanntmachung des Hinweises auf die Patenterteilung: **30.08.2006**
(21) Anmeldenummer: 03023647.5
(22) Anmeldetag: 17.10.2003
(51) Int. Cl.: A61B 17/70

(54) **Element mit einem Schaft und einem damit verbundenen Halteelement zum Verbinden mit einem Stab**
Member having a shaft connected to a holding member for coupling to a rod
Elément ayant une tige et un élément de fixation relié à cet élément pour la couplage avec une barre

(30) Priorität: 02.12.2002 DE 10256095
(43) Veröffentlichungstag der Anmeldung: 09.06.2004
(73) Patentinhaber: BIEDERMANN MOTECH GmbH, 78054 VS-Schwenningen (DE)
(72) Erfinder: Matthis, Wilfried, 79367 Weisweil (DE); Biedermann, Lutz, 78048 VS-Villingen (DE); Harms, Jürgen, 76227 Karlsruhe (DE)
(74) Vertreter: Hofer, Dorothea

(56) Entgegenhaltungen:
- EP-A- 1 219 255
- EP-A- 1 323 391
- DE-A- 19 912 364
- US-B1- 6 368 321

## Beschreibung

Die Erfindung betrifft ein in der Wirbelsäulen- bzw. Unfallchirurgie zu verwendendes Element mit einem Schaft und einem damit verbundenen Halteelement zum Verbinden mit einem Stab nach dem Oberbegriff des Patentanspruchs 1.

Ein derartiges Element ist in Form einer Polyaxial-Knochenschraube aus der EP 0 614 649 B1 bekannt.
Die bekannte Schraube weist zur Sicherung eine auf das Aufnahmeteil aufschraubbare Mutter auf.

Wenn das Gewinde des Aufnahmeteils und der Innenschraube ein metrisches Gewinde ist, wirken beim Einschrauben der Innenschraube Kraftkomponenten in radialer Richtung des zylindrischen Aufnahmeteils, die dazu führen können, daß sich die Schenkel des Aufnahmeteils aufspreizen, wobei es zur Lockerung der Innenschraube kommt.

Es sind Implantate der eingangs beschriebenen Art bekannt, bei denen nur eine Innenschraube zur Stabfixierung verwendet wird, wobei eine spezielle Gewindeform zur Reduzierung der beim Einschrauben radial nach außen wirkenden Kräfte vorgesehen ist. So ist beipielsweise aus der US 5,005,562 eine Monoaxialschraube bekannnt, wobei die Gewindeform von Aufnahmeteil und Innenschraube als Sägengewinde ausgebildet ist, während in der WO 00/27297 die Verwendung eines Gewindes mit negativem Lastflankenwinkel beschrieben ist.

Bei allen zuvor genannten Implantaten mit schmalen Seitenflanken des Aufnahmeteils besteht jedoch bei Verwendung von nur einer Innenschraube zur Fixierung ohne eine zusätzliche Sicherung über eine aufschraubbare Mutter oder eine die Schenkel des Aufnahmeteils außen übergreifende Kappe oder einen Ring oder dergleichen ein Problem, was anhand von Figur 6 beschrieben wird. Diese zeigt schematisch eine Polyaxial-Knochenschraube ähnlich der in der EP 0 614 649 B1 gezeigten. Sie weist ein Schraubenelement 101 mit einem kugelsegmentförmigen Kopf 102 auf, der in einem Aufnahmeteil 103 mit einer U-förmigen Ausnehmung zum Einlegen des Stabs 100 gehalten ist. Auf den kugelsegmentförmigen Kopf 102 wirkt ein Druckelement 104 und zum Fixieren von Stab und Kopf ist eine in das Aufnahmeteil 103 einschraubbare Innenschraube 105 mit einem metrischen Gewinde vorgesehen. Beim endfesten Anziehen der Innenschraube mit hohem Drehmoment kippt diese um die Stabauflagefläche bzw. dreht sich um den Stab, so daß eine Torsionskraft auf die Schenkel des Aufnahmeteils wirkt, die diese gegeneinander verwindet. Dies führt dann zu einer unsymmetrischen Spreizung und einer Verformung der Gewindeaufnahmeteile, wobei dann die Innenschraube aus dem rechten unteren und linken oberen jeweils durch einen Kreis in Fig. 6 dargestellten Gewindegang herausrutschen kann und der betreffende Gewindegang somit übersprungen werden kann.

Um dies zu vermeiden, müßte die Innenschraube mit reduziertem Anzugsmoment angezogen werden, was aber die Haltekraft beeinträchtigt. Ferner kann das Problem auch verringert werden, wenn die Wand des Aufnahmeteils sehr stark dimensioniert wird, was aber dem Erfordernis der kompakten Bauweise von Implantaten entgegensteht.

Das beschriebene Problem des Verkippens der Innenschraube beim endfesten Anziehen ist unabhängig von der verwendeten Gewindeform.

Aus der EP 1 219 255 A1 ist ein Element mit den Merkmalen des Oberbegriffs des Patentanspruchs 1 bekannt. Das Innengewinde der Schenkel weist einen negativen Lastflankenwinkel auf.

Aufgabe der Erfindung ist es, ein Element der eingangs beschriebenen Art zu schaffen, bei dem eine zuverlässige Stabfixierung und gleichzeitig eine kompakte Bauweise möglich ist.

Diese Aufgabe wird durch das in Patentanspruch 1 gekennzeichnete Element gelöst. Weiterbildungen der Erfindung sind in den Unteransprüchen angegegeben.

Die Erfindung ist nicht nur für Polyaxial-Knochenschrauben, sondern auch für Monoaxial-Knochenschrauben oder -haken geeignet und eignet sich für alle Gewindeformen, die für das Aufnahmeteil und die Innenschraube verwendet werden. Die Erfindung ist außerdem besonders vorteilhaft im Hinblick auf die Verwendung eines Rundstabs mit kreisförmigem Querschnitt.

Weitere Merkmale und Zweckmäßigkeiten der Erfindung ergeben sich aus der Beschreibung von Ausführungsbeispielen anhand der Figuren. Von den Figuren zeigen:
- Fig. 1:: eine Schnittansicht einer ersten Ausführungsform mit noch nicht festgezogener Innenschraube;
- Fig. 2:: eine Schnittansicht der ersten Ausführungsform mit festgezogener Innenschraube;
- Fig. 3:: eine schematische Darstellung des Druckelements im Verhältnis zum Stab für die erste Ausführungsform;
- Fig. 4:: eine Schnittansicht einer zweiten Ausführungsform der Erfindung mit noch nicht festgezogener Innenschraube
- Fig.'5:: eine Schnittansicht der zweiten Ausführungsform der Erfindung mit festgezogener Innenschraube; und
- Fig. 6:: eine schematische Schnittdarstellung einer herkömmlichen Polyaxialschraube mit Innenschraube beim endfesten Anziehen der Innenschraube.

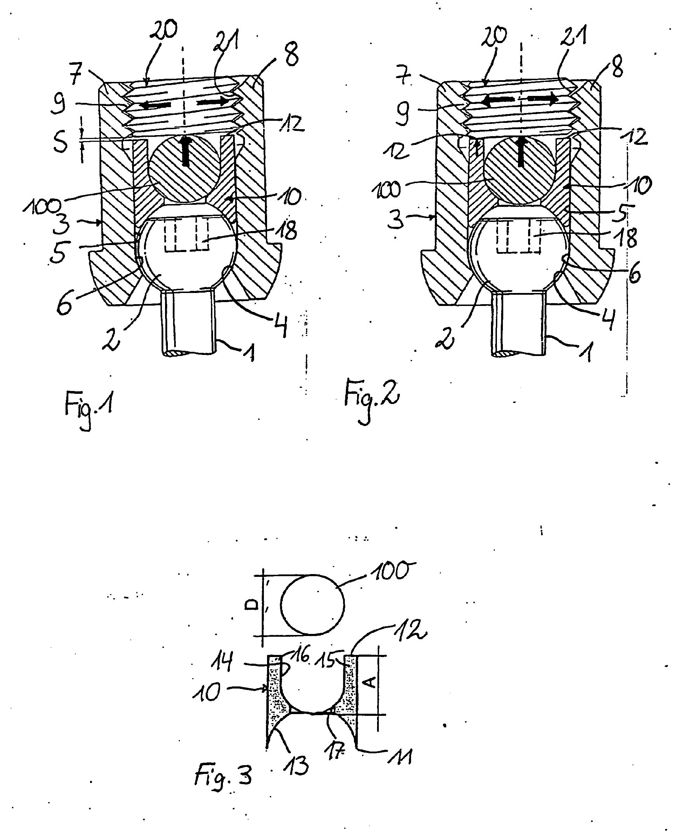

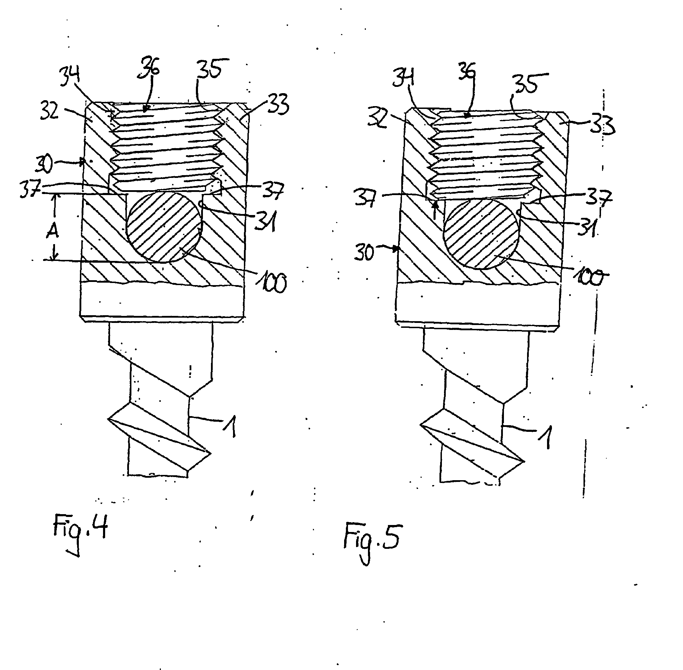

In der in Fig. 1 bis 3 gezeigten Ausführungsform ist das erfindungsgemäße Element als Polyaxialschraube ausgebildet, die ein Schraubenelement mit einem Gewindeschaft 1 mit einem nichtdargestellten Knochengewinde und einem kugelsegmentförmigen Kopf 2 aufweist, der mit einem Aufnahmeteil 3 verbunden ist. Das Aufnahmeteil 3 hat an seinem einen Ende eine axialsymmetrisch ausgerichtete erste Bohrung 4, deren Durchmesser größer als der des Gewindeabschnitts des Schafts 1 und kleiner als der des Kopfes 2 ist. Ferner weist das Aufnahmeteil 3 eine koaxiale zweite Bohrung 5 auf, die auf dem der ersten Bohrung 4 gegenüberliegenden Ende offen ist und deren Durchmesser so groß ist, daß das Schraubenelement durch das offene Ende mit seinem Gewindeabschnitt durch die erste Bohrung 4 hindurch und mit dem Kopf 2 bis zum Grund der zweiten Bohrung 5 führbar ist. Zwischen der ersten und der zweiten Bohrung ist ein kleiner koaxialer Abschnitt 6 vorgesehen, der unmittelbar an die erste Bohrung 4 angrenzt und zum offenen Ende hin sphärisch ausgebildet ist, wobei der Radius im wesentlichen gleich dem kugelsegmentförmigen Abschnitts des Kopfs 2 ist.

Das Aufnahmeteil 3 weist ferner in bekannter Weise eine zur Mitte des Teiles symmetrisch angeordnete U-förmige Ausnehmung zur Aufnahme des Stabs 100 auf, deren Grund zur ersten Bohrung 4 hin gerichtet ist und durch die zwei freie Schenkel 7, 8 gebildet sind. In einem Bereich angrenzend an ihr freies Ende weisen die Schenkel 7, 8 ein Innengewinde 9 auf, das in dieser Ausführungsform als ein metrisches Gewinde ausgebildet ist.

Es ist ferner ein zylindrisches Druckelement 10 mit einem ersten Ende 11, das in einem in das Aufnahmeteil 3 eingesetztem Zustand dem Kopf 2 zugewandt ist, und einem dem ersten Ende gegenüberliegenden zweiten Ende 12 vorgesehen. Der Außendurchmesser des Druckelements ist etwas kleiner als der der Bohrung 5 des Aufnahmeteils, so daß das Druckelement in dem Bereich der Bohrung 5 gleiten kann, also zu dem Kopf 2 hin verschiebbar ist.

Das Druckelement weist ferner an seinem ersten Ende 11 eine sich zu dem Ende hin erweiternde kugelsegmentförmige Ausnehmung 13 auf, deren Kugelradius so gewählt ist, dass er in einem in das Aufnahmeteil eingesetzten Zustand den Kopf 2 teilweise umgreift.

Ferner ist an dem Druckelement 10 an dem der kugelsegmentförmigen Ausnehmung 13 gegenüberliegenden zweiten Ende 12 eine U-förmige Ausnehmung 14 vorgesehen, durch die zwei Schenkel 15, 16 gebildet sind. Die Abmessungen der U-förmigen Ausnehmung 14 des Druckelements ist so bemessen, daß die Ausnehmung einen Kanal bildet, in den der Stab 100 eingelegt werden kann. Wie insbesondere in den Figuren 2 und 3 gezeigt ist, ist die in Richtung der Zylinderachse des Druckelements gesehene Tiefe A der U-förmigen Ausnehmung 14 kleiner als der Durchmesser D des aufzunehmenden Stabs 100. In einem in Fig. 1 gezeigten Zustand, in dem das Druckelement 10 in das Aufnahmeteil eingesetzt ist und der Stab 100 eingelegt ist, ragt somit der Stab 100 um eine Höhe S über das erste Ende 12, das durch das freie Ende der Schenkel 15, 16 gebildet ist, hinaus. Die Höhe S beträgt bei den üblichen Dimensionen der Polyaxialschraube etwa 1% bis 7.5% des Stabdurchmessers D.

Das Druckelement weist ferner eine zentrale Bohrung 17 auf, die sich durch dieses hindurch erstreckt und deren Durchmesser gerade so groß bemessen ist, dass ein Schraubwerkzeug zum Ineingriffbringen mit einer in dem Kopf 2 vorgesehenen Ausnehmung 18 hindurchführbar ist.

Ferner ist eine Innenschraube 20 vorgesehen, die ein mit dem Innengewinde 9 der Schenkel 7, 8 des Aufnahmeteils zusammenwirkendes Außengewinde 21 aufweist und die so bemessen ist, daß sie in eingeschraubtem Zustand, wenn sie auf den eingelegten Stab drückt, nicht oder nicht wesentlich über das Aufnahmeteil hervorsteht. Das Innengewinde 9 des Aufnahmeteils ist vorzugsweise so bemessen, daß es sich ausgehend vom freien Ende der Schenkel 7, 8 nicht tiefer als die Oberkante des Stabes, wenn dieser im Grund der U-förmigen Ausnehmung des eingesetzten Druckelements ruht, erstreckt.

Im Betrieb wird zuerst das Schraubenelement in das Aufnahmeteil 3 eingeführt, bis der Kopf 2 an dem sphärischen Abschnitt 6 anliegt. Dann wird das Druckelement 10 eingesetzt und so positioniert, daß die U-förmige Ausnehmung des Aufnahmeteils und die U-förmige Ausnehmung 14 des Druckelements 10 korrespondieren. In diesem Zustand wird das Schraubenelement über die Ausnehmung 18 in den Knochen eingeschraubt. Dann wird der Stab 100 eingelegt und die Innenschraube eingeschraubt. Wenn die Winkelstellung des Schraubenelements relativ zu dem Aufnahmeteil 3 justiert ist und der Stab 100 justiert ist, wird die Innenschraube 20 angezogen. Anfangs berührt ihre Unterseite den Stab nur leicht, wobei dann zwischen der Unterseite der Innenschraube und dem freien zweiten Ende 12 des Druckelements 10 ein Spalt der Größe S, wie in Fig. 1 gezeigt ist, vorhanden ist. Die Schenkel 7,8 des Aufnahmeteils können dabei eventuell schon etwas auseinandergespreizt werden, was durch die Pfeile angedeutet ist. Beim endfesten Anziehen der Innenschraube und dem Aufbringen hoher Kräfte, versucht diese dem auf sie wirkenden Druck auszuweichen und um den Stab zu kippen, wird aber daran gehindert, weil ihr unterer Rand von dem freien Ende 12 eines der Schenkel 15, 16 des Druckelements 10, welches ein Widerlager bildet, abgestützt wird. Dadurch wird ein Einwirken von Torsionskräften beim endfesten Anziehen vermieden und die Spreizung der Schenkel des Aufnahmeteils verhindert. Deshalb ist es auch möglich höhere Drehmomente beim endfesten Anziehen aufzubringen und damit eine große Haltekraft zu erzeugen, ohne daß eine übermäßige Spreizung oder Verkippung erfolgt. Ein Springen der Innenschraube über einen Gewindegang des Aufnahmeteils tritt nicht mehr auf. Im vollständig eingeschraubten Endzustand fixiert die Innenschraube den Stab und über das Druckelement und den Stab auch den Kopf 2.

Ein wesentlicher Vorteil besteht darin, daß die Wandstärke des Aufnahmeteils geringer ausgeführt werden kann und damit die Bauweise kompakter ist und daß eine hohe Sicherheit gegen ein Verkippen der Innenschraube beim Festziehen gegeben ist.

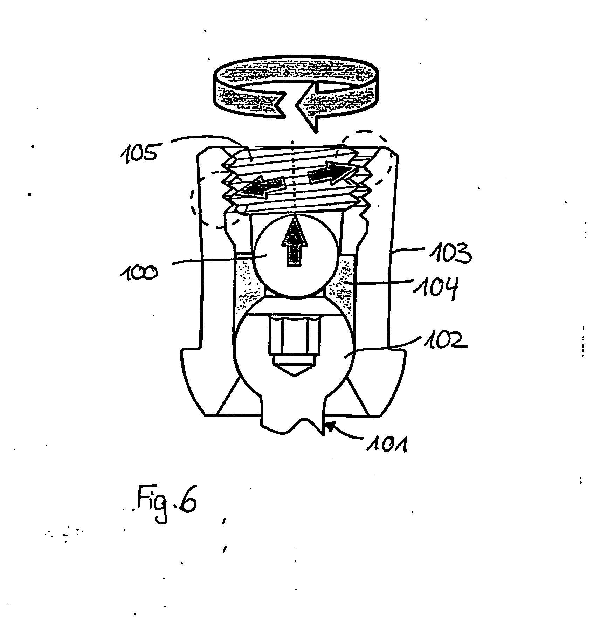

Das erfindungsgemäße Element ist in der in der in den Figuren 4 und 5 dargestellten Ausführungsform als Monoaxial-Knochenschraube ausgebildet. Diese weist einen Schaft 1 mit einem Knochengewindeabschnitt und ein damit starr verbundenes Aufnahmeteil 30 zur Aufnahme des Stabes 100 auf. Hierzu ist das Aufnahmeteil mit einer Ausnehmung 31 mit einem U-förmigen Querschnitt versehen, die gerade so groß bemessen ist, daß der Stab 100 einlegbar ist und in den Grund der Ausnehmung passt. Durch die U-förmige Ausnehmung 31 sind zwei freie Schenkel 32, 33 gebildet, die angrenzend an ihr freies Ende ein Innengewinde 34 aufweisen, welches mit einem entsprechenden Außengewinde 35 einer zwischen die Schenkel einzuschraubenden Innenschraube 36 zum Fixieren des Stabs 100 zusammenwirkt.

Die U-förmigen Ausnehmung 31 hat, wie in Fig. 4 gezeigt ist, von ihrem Grund bis zu einer Höhe A eine Kanalbreite die etwas größer ist, als der Durchmesser D des aufzunehmenden Stabs, so daß dieser in dem durch die Ausnehmung gebildeten Kanal verschiebbar ist. Anschließend an diesen Bereich ab der Höhe A bis zum freien Ende der Schenkel 32, 33 ist der Innendurchmesser des Aufnahmeteils 30 größer als der Durchmesser des Stabes. Somit ist am Übergang zwischen diesen Bereichen an der Innenseite jedes Schenkels 32, 33 eine Schulter bzw. ein Vorsprung 37 gebildet, der ein Widerlager zur Abstützung für die Innenschraube zum Verhindern eines Kippens bildet. Der Vorsprung 37 kann z.B. als der Gewinde-freistich für das Innengewinde mit einer planen Fläche ausgebildet sein.

Der Betrieb und die Funktionsweise ist ähnlich wie bei der ersten Ausführungsform. Die Auflagefläche 37 wirkt, wie in Fig. 5 gezeigt ist, als Abstützung bei einem Verkippen der Innenschraube und setzt dem Rand der Innenschraube eine durch den kleinen Pfeil gekennzeichnete Gegenkraft entgegen, die ein Verwinden der Schenkel beim Anziehen verhindert. Es werden somit dieselben Effekte wie bei der ersten Ausführungsform erzielt.

Abwandlungen der beschriebenen Ausführungsformen sind möglich: So kann anstelle des metrischen Gewindes von Aufnahmeteil und Innenschraube auch ein Sägengewinde, ein Gewinde mit negativem Lastflankenwinkel oder ein Flachgewinde vorgesehen sein. Letzteres zeichnet sich dadurch aus, daß beide Gewindeflanken mit der Schraubenachse einen Winkel von 90° einschließen. Bei den genannten Gewindeformen tritt ein Aufspreizen der Schenkel nicht auf, weil keine radial nach außen gerichteten Kraftkomponenten wirken. Trotzdem kommt es beim endfesten Anziehen der Innenschraube zu einem Verkippen und damit zu Torsionskräften die zu einer Gewindeverformung der Außenflanken des Aufnahmeteils führen. Dies wird durch die Erfindung zuverlässig verhindert.

Die beschriebenen Gewinde können ferner als Rechts- oder Linksgewinde ausgebildet sein.

Die Innenschraube kann auch als hülsenartiges Teil mit einem Außengewinde ausgebildet sein und kann axial mit einem durchgehenden oder nicht durchgehenden Schlitz versehen sein.

Anstelle des Schafts mit dem Knochengewinde kann auch ein Haken vorgesehen sein.

## Patentansprüche

1. Element mit einem Schaft (1) und einem damit verbundenen Halteelement (3; 30) zum Verbinden mit einem Stab (100), wobei das Halteelement (3; 30) eine einen U-förmigen Querschnitt aufweisende Ausnehmung zur Aufnahme des Stabs und zwei an einem Ende freie Schenkel (7, 8; 32, 33), die ein Innengewinde (9; 34) aufweisen, beinhaltet
und mit einem Verschlußelement (20; 36) zum Fixieren des in die U-förmige Ausnehmung eingelegten Stabs, welches ein Außengewinde aufweist, das mit dem Innengewinde der Schenkel zusammenwirkt,
**dadurch gekennzeichnet, daß** an bzw. in dem Halteelement (3; 30) zusätzlich zu den Gewindegängen des Innengewindes eine Sicherungsvorrichtung vorgesehen ist, die beim endfesten Anziehen des Verschlußelements ein Kippen des Verschlußelements um den Stab begrenzt und wobei die Sicherungsvorrichtung durch eine Auflagefläche (12; 37) für einen Abschnitt der dem Grund der U-förmigen Ausnehmung zugewandten Unterseite des Verschlußelements (20; 36) gebildet ist, und die Auflagefläche ein Widerlager für das Verschlußelement beim Kippen bildet.

2. Element nach Anspruch 1, **dadurch gekennzeichnet, daß** der Abstand (A) zwischen dem Grund der U-förmigen Ausnehmung bis zu der Auflagefläche (12; 37) kleiner ist, als der Durchmesser (D) des einzulegenden Stabs.

3. Element nach Anspruch 2, **dadurch gekennzeichnet, daß** der Abstand (A) um etwa 1% bis 7.5% des Durchmessers (D) kleiner ist, als der Durchmesser (D).

4. Element nach einem der Ansprüche 1 bis 3, **dadurch gekennzeichnet, daß** die Sicherungsvorrichtung als ein an der Innenseite der freien Schenkel (32, 33) des Halteelements (30) vorgesehener Vorsprung (37) ausgebildet ist.

5. Element nach einem der Ansprüche 1 bis 4, **dadurch gekennzeichnet, daß** der Schaft (1) und das Halteelement(30) monoaxial verbunden, bevorzugt einstückig, ausgebildet sind.

6. Element nach einem der Ansprüche 1 bis 3 , **dadurch gekennzeichnet, daß** der Schaft (1) an einem Ende einen Kopf (2) aufweist, der mit dem Halteelement polyaxial verbunden ist, und daß ein auf den Kopf einwirkendes Druckelement (20) vorgesehen ist zum Fixieren der Winkelstellung und wobei die Sicherungsvorrichtung an dem Druckelement (20) vorgesehen ist.

7. Element nach Anspruch 6, **dadurch gekennzeichnet, daß** das Druckelement (20) eine zu der Ausnehmung des Halteelements (3) korrespondierende U-förmige Ausnehmung (14) aufweist und daß die Sicherungsvorrichtung durch den freien Rand (12) der durch die Ausnehmung gebildeten Schenkel (15, 16) gebildet ist.

8. Element nach Anspruch 7, **dadurch gekennzeichnet, daß** der Abstand (A) vom Grund der U-förmigen Ausnehmung zum freien Ende (12) des Druckelements kleiner ist, als der Stabdurchmesser (D).

9. Element nach einem der Ansprüche 1 bis 8, **dadurch gekennzeichnet, daß** das Innengewinde der Schenkel (7, 8; 32, 33) und das Außengewinde des Verschlußelements (20; 36) als metrisches Gewinde oder als Sägengewinde oder als Gewinde mit einem negativen Lastflankenwinkel oder als Flachgewinde ausgebildet ist.

## Claims

1. Element with a shaft (1) and with a holding element (3; 30) connected thereto for connecting to a rod (100),
wherein the holding element (3; 30) includes a recess having a U-shaped cross-section for receiving the rod and two legs (7, 8; 32 ,33) free at one end which comprise an inner thread (9; 34);
and with a closure element (20; 36) for fixing the rod inserted into the U-shaped recess, the closure element having an outer thread cooperating with the inner thread of the legs;
**characterized in that**
on or in the holding element (3; 30) additionally to the thread turns of the inner thread a securing device is provided which, at the time of final tightening of the closure element, limits tilting of the closure element about the rod and
wherein the securing device is formed by a support surface (12; 37) for a section of the lower side of the closure element (20; 36) facing the bottom of the U-shaped recess, and the support surface forms an abutment for the closure element when tilting.

2. Element according to claim 1, **characterized in that** the distance (A) between the bottom of the U-shaped recess to the support surface (12; 37) is smaller than the diameter (D) of the rod to be inserted.

3. Element according to claim 2, **characterized in that** the distance (A) is smaller than the diameter (D) by about 1% to 7.5% of the diameter (D).

4. Element according to one of claims 1 to 3, **characterized in that** the securing device is formed as a projection (37) provided on the inner side of the free legs (32, 33) of the holding element (30).

5. Element according to one of claims 1 to 4, **characterized in that** the shaft (1) and the holding element (30) are formed monoaxially connected, preferably in one piece.

6. Element according to one of claims 1 to 3, **characterized in that**
the shaft (1) at one end has a head (2) which is polyaxially connected to the holding element and that
a pressure element (20) acting upon the head is provided for fixing the angular position, and wherein
the securing device is provided on the pressure element (20).

7. Element according to claim 6, **characterized in that**
the pressure element (20) comprises a U-shaped recess (14) corresponding to the recess of the holding element (3) and that
the securing device is formed by the free edge (12) of the legs (15, 16) which are formed by the recess.

8. Element according to claim 7, **characterized in that** the distance (A) from the bottom of the U-shaped recess to the free end (12) of the pressure element is smaller than the diameter (D) of the rod.

9. Element according to one of claims 1 to 8, **characterized in that** the inner thread of the legs (7, 8; 32, 33) and the outer thread of the closure element (20; 36) are formed as a metric thread or as a saw tooth thread or as a thread having a load flank with a negative angle or as a flat thread.

## Revendications

1. Elément avec une tige (1) et un élément de fixation (3 ; 30) relié à celle-ci pour la liaison à une barre (100), l'élément de fixation (3 ; 30) présentant un évidement à section transversale en forme de U pour recevoir la barre et deux branches (7, 8 ; 32 , 33) libres à son extrémité, présentant un filetage intérieur (9 ; 34), et avec un élément de fermeture (20 ; 36) pour la fixation de la barre introduite dans l'évidement en forme de U, qui présente un filetage extérieur, coopérant avec le filetage intérieur des branches,
**caractérisé en ce que**, sur ou dans l'élément de fixation (3 ; 30), en plus des pas de vis du filetage intérieur, est prévu un dispositif de sécurité qui, lors du serrage final à bloc de l'élément de fermeture, limite un basculement de l'élément de fermeture autour de la barre, et le dispositif de sécurité étant formé par une face de pose (12 ; 37) pour un tronçon de la face inférieure, tournée vers le fond de l'évidement en forme de U, de l'élément de fermeture (20 ; 36), et la face de pose formant un contre-appui pour l'élément de fermeture lors du basculement.

2. Elément selon la revendication 1, **caractérisé en ce que** l'espacement (A) entre le fond de l'évidement en forme de U, jusqu'à la face de pose (12 ; 37), est inférieur au diamètre (D) de la barre à insérer.

3. Elément selon la revendication 2, **caractérisé en ce que** l'espacement (A) est inférieur au diamètre (D), d'une valeur d'environ 1 % à 7, 5 % du diamètre (D).

4. Elément selon l'une des revendications 1 à 3, **caractérisé en ce que** le dispositif de sécurité est réalisé sous la forme d'une saillie (37), prévue sur la face intérieure des branches (32 ; 33) libres de l'élément de maintien (30).

5. Elément selon l'une des revendications 1 à 4, **caractérisé en ce que** la tige (1) et l'élément de fixation (30) sont reliés de façon mono-axiale, de préférence en étant réalisés d'une seule pièce.

6. Elément selon l'une des revendications 1 à 3, **caractérisé en ce que** la tige (1) présente, sur une extrémité, une tête (2) reliée de façon polyaxiale à l'élément de fixation, et **en ce qu'**un élément de pressage (20) agissant sur la tête est prévu, pour fixer la position angulaire et le dispositif de sécurité étant prévu sur l'élément de pressage (20).

7. Elément selon la revendication 6, **caractérisé en ce que** l'élément de pressage (20) présente un évidement (14) en forme de U, correspondant à l'évidement de l'élément de fixation (3), et **en ce que** le dispositif de sécurité est formé par le bord (12) libre des branches (15 ; 16) formées par l'évidement.

8. Elément selon la revendication 7, **caractérisé en ce que** l'espacement (A) depuis le fond de l'évidement en forme de U jusqu'à l'extrémité (12) libre de l'élément de pressage est inférieur au diamètre de barre (D).

9. Elément selon l'une des revendications 1 à 8, **caractérisé en ce que** le filetage intérieur des branches (7, 8 ; 32, 33) et le filetage extérieur de l'élément de fermeture (20 ; 36) sont réalisés sous forme de filetage métrique, ou de filetage en dents de scie, ou en tant que filetage avec un angle de flanc de charge négatif, ou en tant que filetage plat.
